Europäisches Patentamt

European Patent Office

Office européen des brevets

(19)

(11) Numéro de publication : **0 086 707**
**B1**

(12) **FASCICULE DE BREVET EUROPÉEN**

(45) Date de publication du fascicule du brevet :
07.09.88

(21) Numéro de dépôt : 83400274.3

(22) Date de dépôt : 08.02.83

(51) Int. Cl.⁴ : **C 12 N 15/00, C 07 H 21/04, C 12 P 21/02, C 07 K 1/00, A 61 K 39/13**

(54) **Fragments d'ADN codant pour un peptide immunogène susceptible d'induire in vivo la synthèse d'anticorps anti-poliovirus.**

(30) Priorité : 08.02.82 FR 8202013

(43) Date de publication de la demande :
24.08.83 Bulletin 83/34

(45) Mention de la délivrance du brevet :
07.09.88 Bulletin 88/36

(84) Etats contractants désignés :
BE CH DE GB LI

(56) Documents cités :
EP-A- 0 065 924
WO-A-82 /036 32
PROC. NATL. ACAD. SCI. USA, vol. 78, no. 10, octobre 1981, pages 5983-5987, US. S. VAN DER WERF et al.: "Molecular cloning of the genome of poliovirus type 1"
PROC. NATL. ACAD. SCI. USA, vol. 78, no. 8, août 1981, pages 4887-4891, US. V.R. RACANIELLO et al.: "Molecular cloning of poliovirus cDNA and determination of the complete nucleotide sequence of the viral genome"

(73) Titulaire : INSTITUT PASTEUR
25-28, rue du Docteur Roux
F-75724 Paris Cédex 15 (FR)

(72) Inventeur : Girard, Marc
6, rue César Franck
F-75015 Paris (FR)
Inventeur : van der Werf, Sylvie
3, Villa Grenelle
F-75015 Paris (FR)

(74) Mandataire : Gutmann, Ernest et al
S.C. Ernest Gutmann - Yves Plasseraud 67, boulevard Haussmann
F-75008 Paris (FR)

## Description

L'invention est relative à un fragment d'ADN codant pour un peptide immunogène susceptible d'induire in vivo la formation d'anticorps anti-poliovirus. Elle concerne plus particulièrement des fragments d'ADN de ce type ayant, au niveau de l'information génétique qu'ils contiennent, des parties en commun avec les poliovirus, de préférence, mais non exclusivement du type PV-1, tout en étant cependant de tailles plus réduites. En d'autres termes, l'invention concerne un fragment d'ADN codant pour un déterminant antigénique appartenant normalement aux peptides codés par l'ARN du poliovirus ou par des cADN correspondants, ce déterminant antigénique jouant un rôle essentiel au niveau des propriétés antigéniques des produits d'expression de l'ARN viral naturel.

Les fragments d'ADN selon l'invention possèdent une longueur n'excédant pas celle d'un fragment d'ADN comportant de l'ordre de 1,2 kb (kilopaires de bases), ces fragments étant plus particulièrement caractérisés en ce qu'ils contiennent une séquence nucléotidique codant pour la protéine VP1 ou pour la partie de cette dernière qui code pour celui ou ceux des déterminants antigéniques qui peuvent être considérés comme essentiellement responsables de l'immunogénicité et de la spécificité immunologique de la protéine VP-1 du poliovirus.

Ce résultat est d'autant plus remarquable qu'il va à l'encontre des enseignements de l'état de la technique. Selon B. MANDEL (Advances in virus research, vol. 23) il était admis que la particule infectieuse était nécessaire à l'induction d'anticorps neutralisant le poliovirus. Cette hypothèse a été renforcée par les résultats de MELOEN et Coll. (J. Gen. Virol., 45, 1979, p. 761-763), qui n'ont pas obtenu des anticorps neutralisants avec des polypeptides structuraux séparés par électrophorèse en gel de polyacrylamide-SDS.

L'invention concerne également les vecteurs, notamment plasmides ou phages, qui contiennent dans un de leurs sites de restriction spécifiques l'un des fragments tels que ci-dessus définis, fragments hétérologues vis-à-vis de ce qui peut alors être considéré comme le « corps » du vecteur (séquence(s) d'ADN propre(s) au vecteur lui-même).

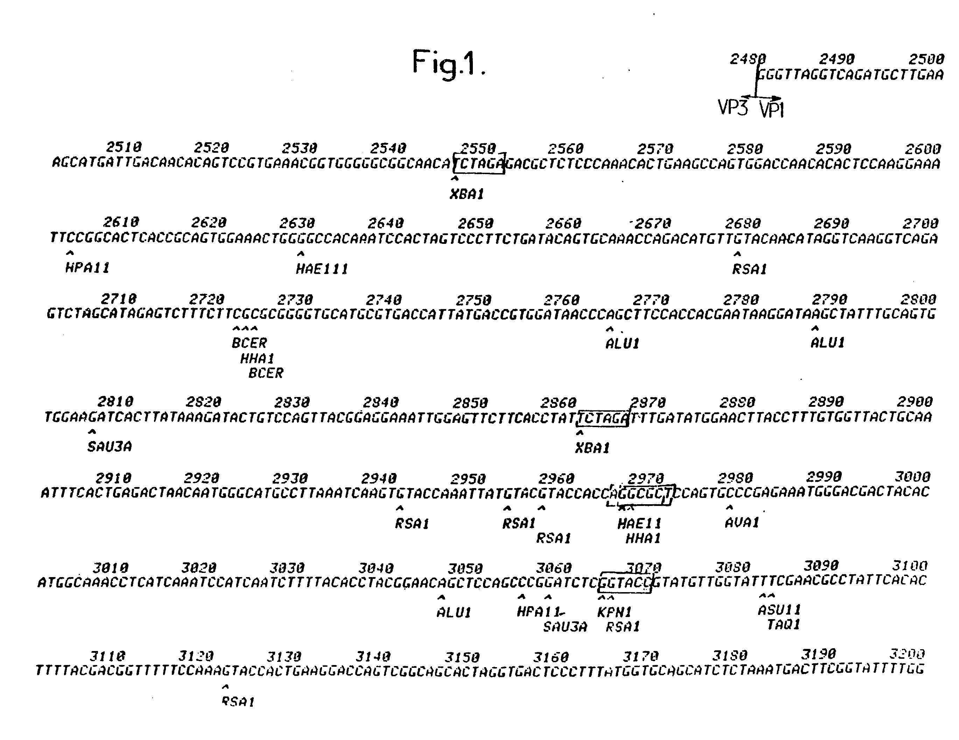

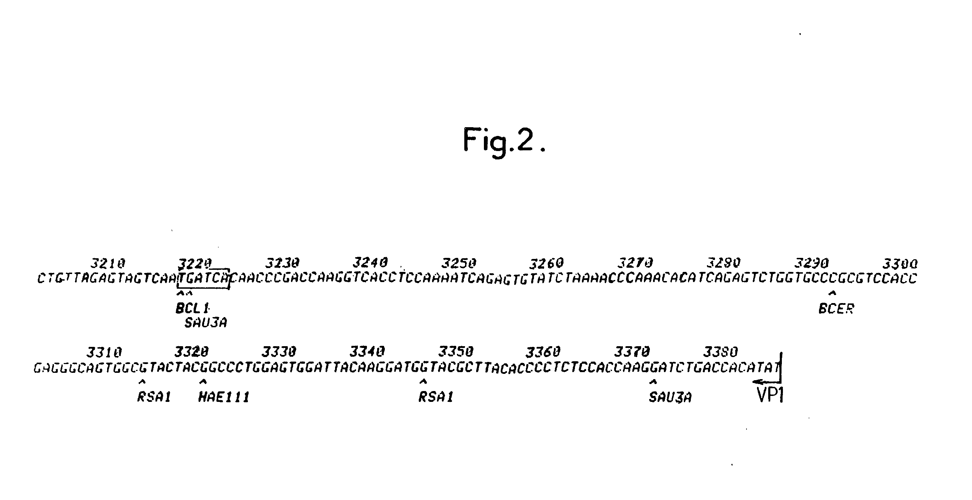

L'invention découle de la découverte que le peptide ou fragment de protéine codé par le fragment d'ADN représenté dans la succession des figures 1 et 2 ci-annexées, est susceptible de former des complexes antigène-anticorps avec des sérums neutralisants mono ou polyclonaux, préparés par injection à l'animal du poliovirus entier fixé au formol (sérum de spécificité D).

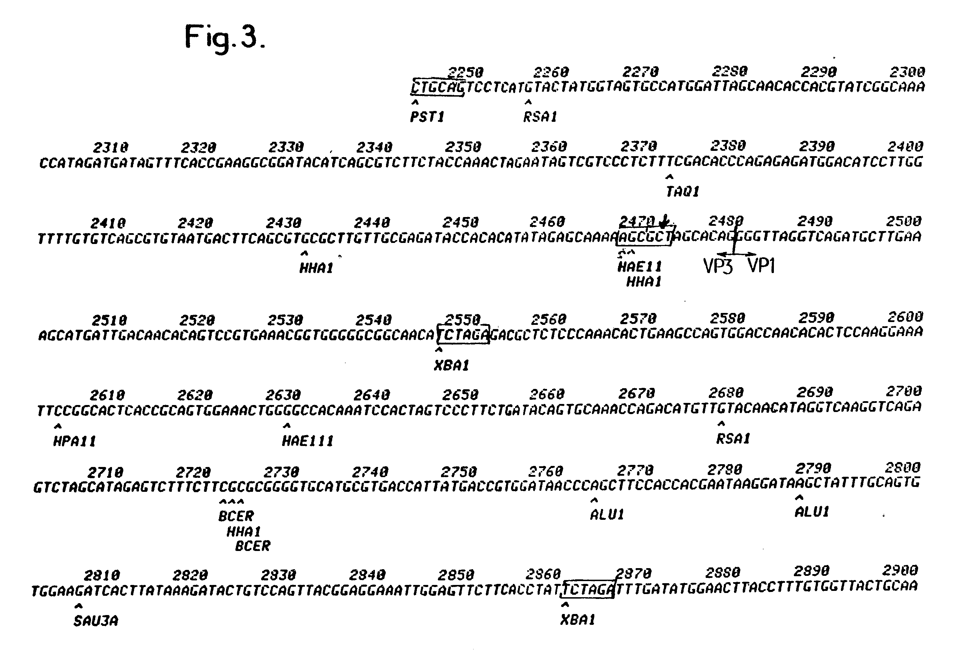

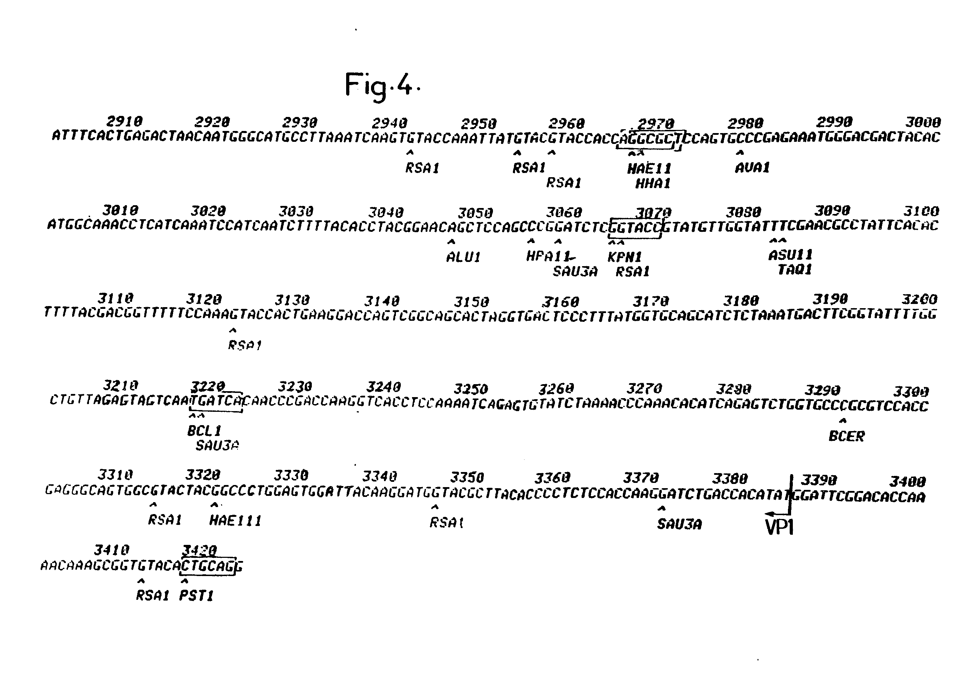

L'invention concerne bien entendu également des fragments d'ADN qui peuvent être un peu plus grands, par exemple celui qui est normalement délimité par des sites PstI aux positions 2 243 et 3 417 (vis-à-vis de l'extrémité 5'), dans le fragment d'ADN clonable du poliovirus décrit dans l'article de Sylvie Van Der WERF et autres auteurs intitulé « Molecular cloning of the genome of poliovirus » dans Proc. Nat. Acad. Sci. U.S.A., volume 78, n° 10, pp. 5 983-5 987, octobre 1981. La séquence de nucléotides de ce fragment d'ADN est indiquée dans la succession des figures 3 et 4.

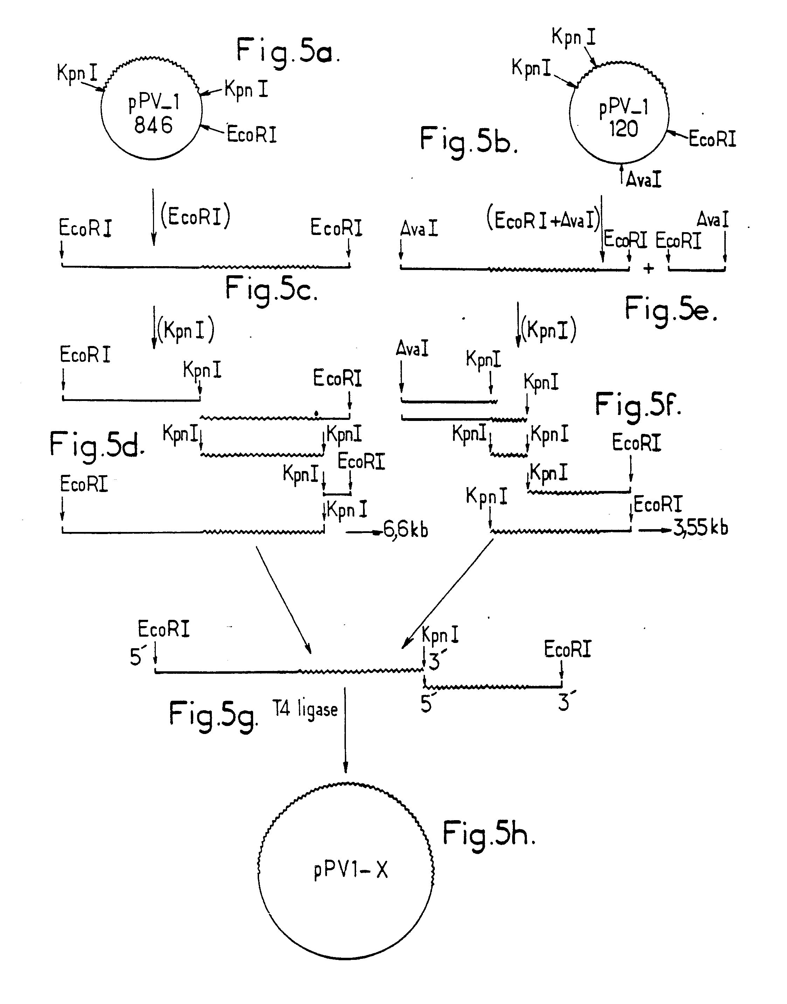

La séquence de nucléotides selon l'invention codant la protéine VP1 peut être obtenue à partir d'un précurseur plus courant la contenant. Les figures 5a à 5h schématisent un mode de production d'un tel précurseur, obtenu à partir des clones pPV1-846 et pPV1-120 décrits dans l'article sus-indiqué. Le plasmide PV1-846 a été déposé à la C.N.C.M. sous le n° I-155 et le plasmide 120 sous le n° I-156 le 19 mai 1981. La procédure de recombinaison des deux clones sus-indiqués pour obtenir le clone pPV1-X est décrite ci-après.

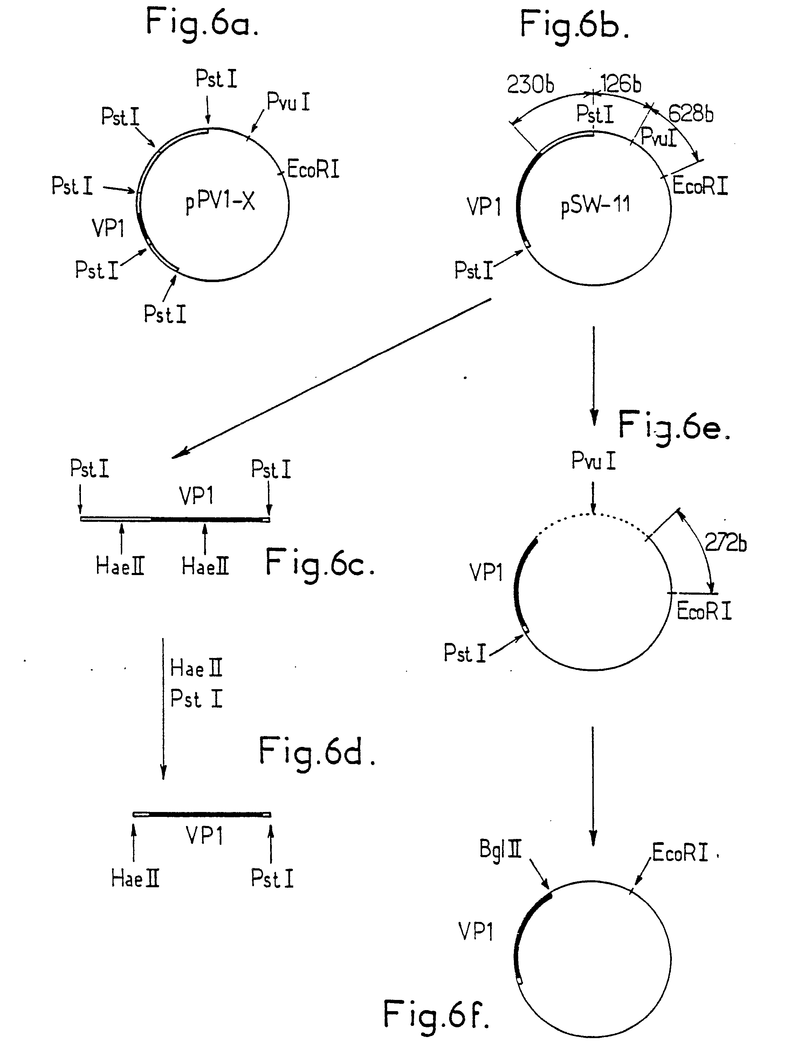

Les figures 6a à 6f représentent schématiquement les étapes d'un mode de production d'un plasmide contenant l'essentiel de l'information génétique de la séquence d'ADN, telle qu'elle résulte des figures 1 et 2.

1. Hydrolyse des DNA clonés par des enzymes de restriction

1.1. L'ADN du plasmide pPV1-846 est hydrolysé complètement par EcoRI. La forme linéaire du DNA plasmidique ainsi obtenue (figure 5c) est hydrolysée par digestion partielle avec Kpn I ; les fragments obtenus (figure 5d) sont séparés par électrophorèse en gel d'agarose à 0,7 %.

Le fragment de taille 6,6 kbp est sélectionné. Il représente en effet la séquence du plasmide pBR322 du site EcoRI au site Pst I, prolongée de celle du DNA correspondant à la séquence du poliovirus qui s'étend du nucléotide 1 ± 35) au nucléotide 3 050 (2ᵉ site Kpn I).

1.2. L'ADN du clone pPV1-120 est hydrolysé dans une digestion complète par AvaI et EcoRI formant ainsi 2 fragments de tailles différentes (figure 5e). L'ADN est hydrolysé ensuite partiellement par Kpn I. Les fragments ainsi obtenus (figure 5f) sont séparés par électrophorèse en gel d'agarose à 0,7 %.

Le fragment de taille 3,55 kbp est sélectionné. Il représente en effet la séquence du cDNA du poliovirus allant du nucléotide 3 050 (2ᵉ site Kpn I) au nucléotide 5 650 environ, prolongée de celle des 752 paires de bases du segment Pst I-EcoRI du plasmide pBR322.

2. Extraction des fragments de DNA des gels

2.1. Les fragments sont visualisés dans les gels par coloration au bromure d'éthidium ; ceux de la taille voulue sont extraits des gels par électroélution en sac à dialyse.

2.2. Le matériel ainsi obtenu est purifié et concentré.

### 3. Recollage des fragments (recombinaison)

Les deux fragments sélectionnés provenant des clones pPV1-846 et pPV1-120 et décrits ci-dessus sont mélangés et recollés à l'aide de la DNA ligase du phage T4. Les bouts collants formés aux points de coupure par EcoRI et KpnI et portés à chaque extrémité des deux fragments favorisent leur recollage et assurent que ce recollage ne se fasse que dans le sens désiré (figures 5g et 5h).

Le génome du plasmide pBR322 est ainsi reconstitué sans modification ni délétion dans le plasmide recombinant. En particulier, les régions nécessaires à sa réplication et à l'expression de la résistance à la tétracycline ne sont pas touchées.

### 4. Transformation de la souche E. coli 1 106

Les fragments des plasmides pPV1-846 et -120 recollés par leurs sites Kpn I et EcoRI sont mises en contact avec des bactéries compétentes de souche E. coli 1 106 dans les conditions de transformation. Les colonies de bactéries résistantes à la tétracycline et sensibles à l'ampicilline sont sélectionnées.

### 5. Analyse des nouveaux clones

5.1. Le DNA plasmidique des bactéries tétracycline résistantes est purifié. Sa masse est déterminée par électrophorèse en gel d'agarose. Elle est égale à celle du plasmide pBR322 augmentée des 5 650 paires de bases du cDNA viral formé par recombinaison.

5.2. L'hybridation in vitro des cDNA ainsi obtenus avec des sondes spécifiques provenant des clones pPV1-846 et pPV1-120 permet la vérification de la présence dans un seul clone recombinant du matériel génétique du poliovirus inséré originellement dans les deux clones parentaux.

5.3. L'analyse détaillée des nouveaux clones est effectuée par les méthodes utilisées antérieurement pour l'étude des clones déjà caractérisés (cartographie physique par enzymes de restriction, microscopie électronique, séquence nucléotidique, etc.).

5.4. Le plasmide pPV1-X obtenu contient dans son insérat la séquence nucléotidique qui code pour les protéines VPO (nucléotides 743 à 1 765), VP3 (nucléotides 1 766 à 2 479) et VP1 (nucléotides 2 480 à 3 385) suivie de la séquence codant pour la protéine P2 (nucléotides 3 386 à environ 5 100) et le début de celle de la protéine P3.

Partant du plasmide pPV1-X, on peut alors obtenir un fragment de cDNA codant pour VP1 en procédant comme suit.

### I. Isolement et reclonage d'un fragment de cDNA renfermant la séquence de VP1

La séquence de nucléotides qui code pour la protéine VP1 est encadrée, dans le génome viral, et par conséquent aussi dans l'insérat porté par pPV1-X, de deux sites PstI, situés respectivement 237 nucléotides en amont (position 2 243) et 32 nucléotides en aval (position 3 417) du premier et du dernier nucléotide de cette séquence (cf. carte de restriction dans la susdite publication et fig. 1 et 2).

Le découpage de pPV1-X (fig. 6a) par l'enzyme de restriction PstI engendre donc une famille de fragments ayant des longueurs correspondant respectivement à 4,36 kb (corps du plasmide) et à 1,8 kb ; 0,43 kb ; 1,17 kb et environ 2,23 kb. Le fragment de 1,17 kb porte la séquence nucléotidique codant pour la fin de VP3 et la totalité de VP1. Ce dernier fragment commence par la séquence nucléotidique : $_5$GTCCTCATGTA et s'achève par la séquence GTACACTGCA$_3$. On le sépare des autres fragments PstI par électrophorèse en gel d'agarose. On prélève la bande du gel qui le contient, et on la soumet à une électroélution pour en extraire le DNA. On suit l'électroélution par illumination aux ultraviolets après coloration du gel au bromure d'éthidium. Le fragment ainsi préparé correspond aux nucléotides du poliovirus 2 248 à 3 421. On l'insère par ligation avec de la DNA ligase au site PstI du plasmide vecteur pBR-322 préalablement linéarisé par cette même enzyme. Les plasmides recombinants qu'on a formés ainsi sont clonés dans la souche 1 106 d'Escherichia coli (sélection des colonies devenues résistantes à la tétracycline mais demeurées sensibles à l'ampicilline après transformation par le plasmide).

L'analyse de leur ADN par cartographie aux enzymes de restriction permet d'identifier et de sélectionner les plasmides recombinants qui portent le fragment du cADN polioviral inséré dans le sens contraire aux aiguilles d'une montre par rapport à la carte de pBR-322, c'est-à-dire dans le même sens transcriptionnel que le gène de la β-lactamase (gène de la résistance à l'ampicilline). Il faut noter que l'insertion du fragment 2 248-3 421 au site PstI de pBR-322 interrompt la continuité de la séquence nucléotidique, et inactive donc le gène de la β-lactamase du vecteur, mais ne permet cependant pas d'assurer l'expression des protéines poliovirales car elle entraîne un changement de phase de lecture de l'insérat.

On dénomme pSW-11 (fig. 6b), le plasmide ayant ces propriétés.

### II. Elimination des séquences codant pour la partie C terminale de VP3 : émondage de VP1

Le plasmide pSW-11 contient 232 nucléotides de cADN de poliovirus relevant de la séquence de VP3 en amont de la séquence de VP1, dans le sens transcriptionnel 5 → 3'. Ces nucléotides excédentaires peuvent être enlevés d'au moins deux manières :

a) par traitement ménagé du fragment PstI (préalablement extrait de pSW-11 : fig. 6c) de 1,17 kb par l'enzyme de restriction HaeII (digestion partielle au nucléotide 2 472), puis sélection par électrophorèse du fragment HaeII-PstI de 0,945 kb (fig. 6d) (nucléotides polioviraux 2 472 à 3 421) et reclonage de ce fragment dans les plasmides appropriés. On peut faciliter le reclonage en accrochant de façon en soi connue aux extrémités du fragment émondé des adaptateurs (« linkers ») synthétiques, courtes séquences de nucléotides contenant un site de restriction déterminé obtenu par synthèse, par exemple selon la technique décrite par R. H. SCHELLER et al., Science, tome 196 (1977), pp. 177-180. Le type de « linker » choisi dépend essentiellement du site de coupure de l'enzyme de restriction utilisée dans le vecteur d'expression.

b) par linéarisation du plasmide pSW-11 par digestion complète par l'enzyme Pvul, suivie d'un traitement exonucléolytique à l'enzyme Bal 31, puis de l'addition d'adaptateurs (« linkers ») synthétiques types (Biolabs, Collaborative Research) et de la recircularisation du plasmide par la DNA ligase.

On traite les plasmides ainsi formés par l'enzyme de restriction correspondant au « linker ».

On ouvre donc les molécules. On analyse par migration en électrophorèse en gel d'agarose leurs tailles pour identifier celles qui ont perdu environ 700 paires de base (perte qui dans la figure 6e est symbolisée par un arc de cercle en pointillés), c'est-à-dire quelques 350 de part et d'autre du site Pvul, soit le fragment Pvul-Pstl de pBR-322 plus la séquence de VP3 jusqu'à VP1, d'un côté, et à une longueur similaire de pBR-322 allant de Pvul vers EcoRI, de l'autre côté.

De cette façon, on peut isoler un fragment dont l'une des extrémités coïncide avec l'extrémité de la séquence d'ADN codant pour VP1 ; ou en tous les cas en est très proche.

En effet, le site Pvul se trouvait à 126 paires de bases (b) du site proximal Pstl de la séquence du fragment Pstl de 1,17 kb et à 356 paires de bases de l'extrémité proximale du fragment de cDNA codant pour VP1, dans le plasmide pSW-11.

Après fixation aux extrémités du fragment sélectionné de « linkers » contenant par exemple un site Bglll au moyen d'une ligase, on sélectionne les plasmides dont la taille est de 4,8 à 5 kb (fig. 6f). On détermine ensuite ceux des plasmides qui ont conservé la séquence entière de VP1, tout en ayant perdu la totalité ou la quasi-totalité de VP3. On vérifie cela en déterminant la séquence nucléotidique du fragment Bglll-Pstl des plasmides sélectionnés. Si l'on utilise la méthode de SANGER, on insère les fragments à séquencer dans la forme réplicative du phage M13 et on clone les phages recombinants ainsi constitués. On utilise ensuite leur ADN pour séquencer le fragment inséré, selon la technique décrite par SANGER. On peut aussi procéder à la détermination de la séquence nucléotidique conservée par la méthode décrite par MAXAM et GILBERT.

III. Insertion du fragment émondé dans un vecteur d'expression

La séquence codant pour VP1 ne comporte ni codon d'initiation, ni codon de terminaison. Elle ne comporte non plus ni promoteur pour sa transcription, ni signal de reconnaissance par les ribosomes (séquence de SHINE et DALGARNO, décrite dans GIRARD et HIRTH, Virologie Moléculaire, Edition Doin 1980, pp. 15-46 et 263-264). Pour la faire exprimer, il faut donc l'insérer en phase au milieu de la séquence nucléotidique (et en tout cas derrière l'AUG d'initiation) d'un gène cloné avec son promoteur (ou auquel on adjoindra en amont, un promoteur étranger). L'utilisation de « linkers », comme décrit ci-dessus, permet d'envisager l'utilisation de plusieurs types de vecteurs d'expression différents selon le promoteur considéré : par exemple du type de ceux indiqués ci-après à titre d'exemple.

a) Promoteurs bactériens

C'est notamment le cas des plasmides contenant la région promoteur-opérateur de l'opéron lactose d'E. coli (opéron lac), suivie de la partie 5' du gène de la β-galactosidase. Ces vecteurs, du type pPC (CHARNAY et al, Nucleic Acide Research 1978, tome V, pp. 4 479-4 494) permettent l'insertion du fragment VP1 au site EcoRI situé à 21 nucléotides derrière l'AUG d'initiation de la β-galactosidase. La protéine à laquelle ils donnent naissance comporte donc pour extrémité N terminale les sept (ou huit) premiers acides aminés de la β-galactosidase bactérienne, suivis des acides aminés de VP1.

b) Promoteurs de phage

C'est notamment le cas des plasmides contenant la région promoteur-opérateur de l'opéron de gauche ($P_L$) ou de l'opéron de droite ($P_R$) du phage λ. Ces vecteurs, respectivement du type pKC30 (ROSENBERG, Nature 1981, Vol. 292, p. 128) ou pRL447 (ZABEAU ; dérivé de pRC5 et de pLG400, ce dernier étant décrit dans Cell, 1980, Vol. 20, pp. 543-553) permettent d'effectuer l'insertion du fragment VP1 au sein des séquences nucléotidiques codant respectivement pour l'extrémité N terminale du produit du gène N ou pour celle du produit du gène cro déposé le 8 février 1982 à la C.N.C.M. sous le n° 1-184. Ces systèmes de vecteurs sont propagés à 30 °C dans des bactéries lysogénisées par un phage λ à répresseur thermosensible (cl 857) ou en présence de plasmides porteurs du même gène (cl 857) codant pour un répresseur thermosensible. Ils demeurent inactifs, du fait du répresseur, tant que la culture est maintenue à 30 °C. Le transfert de la culture à 42 °C est suivi de l'activation des promoteurs de λ($P_L$ ou $P_R$) portés par le plasmide recombinant, du fait de l'inactivation du répresseur du gène cl 857.

c) Promoteurs viraux

C'est en particulier le cas de l'utilisation du virus SV40 comme vecteur. Celui-ci a été avantageusement excisé à partir du clone obtenu à partir du recombinant résultant de l'introduction préalable de SV40 dans le site BamH1 d'un plasmide, tel que pML2 ou pBR327, après délétion des sites EcoRi et AccI (à la position 651) que portaient auparavant lesdits plasmides. Dans ce cas, on utilise le promoteur viral tardif et l'on insère le fragment VP1 du poliovirus à la place de tout ou partie de la région codant pour les protéines tardives de SV40 (VP1 ou VP2). On construit ainsi des ADN de SV40 substitués dans lesquels les séquences codant pour les protéines de capside de ce virus sont remplacés par la séquence codant pour la protéine VP1 du poliovirus. Ainsi, l'insertion du fragment HaeII-PstI de poliovirus décrit au paragraphe 3 ci-dessus, à la place du fragment tardif HaeII-PStI de SV40 (nucléotides de 767 à 1 923) aboutit, après remise en phase des deux séquences au niveau du site HaeII, à créer un gène chimérique possédant la séquence de VP1 du poliovirus directement accrochée derrière la portion N terminale de la protéine VP2 du SV40. De nombreuses autres constructions sont possibles, par exemple par insertion du fragment PstI du poliovirus (fragment de 1,17 kb) au site PstI (nucléotide 1 923) du SV40 ou par insertion du fragment HaeII-PstI à la place des séquences AccI-BamH1 (1 563 à 2 468) du SV40. Tous les SV40 chimériques ainsi constitués sont défectifs. Ils ne peuvent se multiplier qu'en présence d'un virus assistant (par exemple, mutant précoce type ts A30 ou ts A58) qui leur apporte les protéines de capside de SV40.

On peut envisager de procéder à des délétions partielles du virus SV40 utilisé comme vecteur, telles que décrites dans l'article de PAVLAKIS, HIZUKA, GORDEN, SEEBURG et HAMER, dans PNAS 1981, 78, p. 7 398-7 402, ce qui permet d'obtenir un vecteur de plus faible poids moléculaire.

On peut, d'autre part, après avoir inséré la séquence de VP1 de poliovirus au sein du gène VP1 du SV40 (par exemple au site EcoRI), éliminer le reste des séquences nucléotidiques de SV40 qui séparent VP1 du poliovirus de l'AUG de VP1 du SV40 (par exemple par utilisation de l'enzyme Bal31, après ouverture au side AccI). On remarque que la séquence des nucléotides au niveau de l'AUG de VP1 du SV40 est : ATG.GCC... (codons des deux premiers acides aminés de VP1).

Or, la séquence du cDNA polioviral après coupure au niveau du site HaeIII (comme décrit en IIa) est : TA.GCA.CAG.GCC... (codons des trois derniers acides aminés de VP3 et du premier acide aminé de VP1 du poliovirus).

L'addition d'un « linker » EcoRI (BIOLABS) devant cette séquence par la DNA ligase aboutit à la formation d'une séquence nouvelle : CGG.AAT.TCC.GTA.GCA.CAG.GGG... (séquence de VP1). On fait suivre la ligation d'une digestion par l'enzyme EcoRI, puis du remplissage de l'extrémité 3' par la DNA polymérase I d'E. coli en présence des quatre désoxyribonucléosides-triphosphates. Ceci conduit à l'obtention d'une extrémité entièrement bicaténarisée dont la séquence est : AAT.TCC.GTA.GCA.CAG.GGG... (séquence de VP1 du poliovirus). La ligation de cet ADN dans un vecteur SV40, derrière le codon du deuxième acide aminé de VP1 du SV40, permet de recréer une séquence en phase :

ATG.GCC.AAT.TCC.GTA.GCA.CAG.GGG
—SV40— linker —poliovirus—

dans laquelle les nucléotides soulignés (TGGCCA) forment un site Bal1 qui n'existait ni dans le SV40 vecteur ni dans le fragment VP1 de poliovirus inséré, et qui permet donc de sélectionner de manière univoque un recombinant « SV40-poliovirus » dans lequel la séquence du VP1 de poliovirus, précédée de celle de cinq acides aminés, est insérée, en phase, derrière celle des deux premiers acides aminés de VP1 du SV40.

d) Promoteurs de virus animaux portés par des plasmides bactériens

C'est le cas des plasmides portant les promoteurs du gène de la thymidine-kinase du virus de l'herpès (pAGO), du gène de l'antigène HBS du virus de l'hépatite B (pAC-2 ou pAC-14) ou des gènes précoces ou tardifs de l'adénovirus 2, etc. L'insertion du fragment VP1 du poliovirus derrière l'AUG du gène viral cloné avec son promoteur permet d'en assurer l'expression dans la cellule animale (après transfection, micro-injection ou fusion cellules-protoplastes), ou in vitro, la transcription par un extrait de cellules animales (cellules HeLa ou VERO), ce qui conduit à la synthèse de l'ARN messager correspondant.

IV. Détection de l'expression de VP1

L'expression des plasmides recombinants porteurs du fragment VP1 et capables de l'exprimer, c'est-à-dire la synthèse de VP1, est détectée par diverses méthodes d'analyse immunologique mettant en jeu l'emploi d'immunsérums anti-poliovirus :
— sérums neutralisants, mono ou polyclonaux, préparés par injection à l'animal de virus fixé au formol (sérums de spécificité D) ;
— sérums non neutralisants préparés par injection à l'animal de virus dissocié par la chaleur en l'absence de formol, ou de capsides virales vides (sérums de spécificité C) ;
— sérums anti-protéines capsidales (VP0, VP1, VP2 ou VP3) du poliovirus, obtenus par injection à l'animal de protéines capsidales préparées par électroélution après séparation sur gel de polyacrylamide-SDS à partir de virions dissociés au SDS et à la chaleur.

Les sérums sont utilisés en tests d'immunoprécipitation sur extraits bactériens prémarqués à la méthionine $^{35}$S, ou en tests radio-immunologiques sur répliques avec IgG marquées à l'$^{125}$I. Ces tests sont aussi bien utilisables aux extraits de cellules animales.

V. Propriétés de VP1

La protéine VP1 produite selon l'une des méthodes ci-dessus, est purifiée selon une technologie classique telle que par exemple la chromatographie d'affinité.

La protéine VP1, synthétisée dans les diverses conditions décrites, peut être couplée à un « carrier », c'est-à-dire à une protéine naturelle ou un polypeptide synthétique ayant un poids moléculaire suffisant pour que le conjugué formé soit capable d'induire in vivo la production d'anticorps selon les techniques classiques. On peut la faire réagir avec les anticorps anti-poliovirus. Elle est immunogène et inoculée à l'animal ; elle induit la synthèse d'anticorps anti-poliovirus.

Par ailleurs, on peut l'utiliser comme réactif pour le diagnostic et le titrage des anticorps antipoliomyélitiques. La séquence d'ADN selon l'invention peut elle-même être utilisée comme sonde d'hybridation permettant la détection de la présence d'ARN viral ou du cADN correspondant dans un échantillon biologique.

L'invention concerne naturellement toutes les séquences d'ADN équivalentes conduisant à des produits d'expression doués de propriétés immunologiques équivalentes, telles que révélées par exemple par la capacité des anticorps induits par le produit d'expression équivalent à réagir avec le produit d'expression des fragments d'ADN plus particulièrement décrits et vice-versa. En particulier, l'invention s'étend à des séquences d'ADN pouvant différer de celles qui ont été plus particulièrement décrites, par des délétions, additions ou substitutions d'acides nucléiques, pour autant que les propriétés immunologiques des produits d'expression soient équivalentes.

L'invention concerne enfin les produits d'expression eux-mêmes, tels que produits par les micro-organismes appropriés transformables par des vecteurs (plasmides ou phages) comprenant un insérat constitué par l'un des susdits fragments d'ADN ou leurs équivalents.

## Revendications

1. Vecteur recombinant, notamment du type phage ou plasmide, caractérisé en ce qu'il contient un insérat consistant essentiellement en un fragment d'ADN issu d'un cADN correspondant à l'ARN d'un poliovirus, possédant jusqu'à 1,2 kilopaires de bases et contenant une séquence nucléotidique codant pour la protéine VP1 du poliovirus ou pour la partie de cette protéine qui code pour celui ou ceux des déterminants antigéniques qui peuvent être considérés comme essentiellement responsables de l'immunogénicité et de la spécificité immunologique de la protéine VP1 du poliovirus, à savoir la capacité de la protéine ou de la partie de cette protéine à former des complexes antigène-anticorps ou des sérums neutralisants mono- ou polyclonaux, préparés par injection à l'animal du poliovirus entier fixé au formol (sérum de spécificité D).

2. Vecteur recombinant selon la revendication 1, caractérisé en ce que le susdit fragment contient la séquence de nucléotides délimitée par deux sites PstI et s'étendant de la position 2 243 à la position 3 417 du cDNA codant pour un poliovirus du type PV-1.

3. Vecteur recombinant selon la revendication 1, caractérisé en ce que le susdit fragment d'ADN contient la séquence de nucléotides s'étendant de la position 2 248 à la position 3 421 du cDNA codant pour un poliovirus du type PV-1.

4. Vecteur recombinant selon la revendication 1, caractérisé en ce que le susdit fragment d'ADN contient la séquence de nucléotides délimitée par des extrémités HaeII et PstI et s'étendant de la position 2 472 à la position 3 421 du cDNA codant pour un poliovirus du type PV-1.

5. Vecteur recombinant selon la revendication 1, caractérisé en ce que le susdit fragment d'ADN contient la séquence de nucléotides s'étendant de la position 2 480 à la position 3 385 du cDNA codant pour un poliovirus du type PV-1.

6. Vecteur recombinant selon la revendication 5, caractérisé en ce que le promoteur est originaire de l'ADN de SV40.

7. Le produit d'expression de l'insérat produit par un micro-organisme transformable par le vecteur selon la revendication 6.

8. Le principe actif de vaccin contenant le produit d'expression de la revendication 7, couplé à une protéine naturelle ou à un polypeptide synthétique ayant un poids moléculaire suffisant pour que le conjugué formé soit capable d'induire in vivo la production d'anticorps antipoliovirus.

## Claims

1. Recombinant vector, particularly of the phage of plasmid type, characterized by the fact that it contains an insert consisting substantially of a DNA fragment coming from a cDNA corresponding to the RNA of a poliovirus, possessing up to 1.2 kilopairs of bases and containing a nucleotide sequence coding for the poliovirus VP1 protein or for the part of this protein which codes for that or those of the antigenic determinants which can be considered as essentially responsible for the immunogenicity and immunological specificity of the poliovirus VP1 protein, i.e. the capacity of the protein or of the part of this protein to form antigen-antibody complexes or mono or polyclonal neutralizing sera, prepared by injection into an animal of the whole poliovirus fixed with formol (serum of D specificity).

2. Recombinant vector according to claim 1, characterized in that the abovesaid fragment contains the nucleotide sequence bounded by two PstI sites and extending from the 2 243 position to the 3 417 position of the cDNA coding for a PV-1 type poliovirus.

3. Recombinant vector according to claim 1, characterized in that the abovesaid fragment contains the nucleotide sequence extending from the 2 248 position to the 3 421 position of cDNA coding for a PV-1 type poliovirus.

4. Recombinant vector according to claim 1, characterized in that the abovesaid fragment contains the nucleotide sequence bounded by the HaeII and PstI ends and extending from the 2 472 position to the 3 421 position of the cDNA coding for a PV-1 type poliovirus.

5. Recombinant vector according to claim 1, characterized in that the abovesaid fragment contains the nucleotide sequence extending from the 2 480 position to the 3 385 position of the cDNA coding for a PV-1 type poliovirus.

6. Recombinant vector according to claim 5, characterized in that the promoter comes from SV40 DNA.

7. Expression product of the insert produced by a micro-organism transformed by the vector according to claim 6.

8. Active principle of vaccine containing the expression product of claim 7, coupled with a natural protein or a synthetic polypeptide having a sufficient molecular weight for the conjugate formed to be capable of inducing in vivo the production of antipoliovirus antibodies.

**Patentansprüche**

1. Rekombinationsvektor, insbesondere vom Phagen- oder Plasmidtyp, dadurch gekennzeichnet, daß er ein Insert enthält, das im wesentlichen aus einem DNS-Fragment besteht, welches aus einer der RNS eines Poliovirus entsprechenden cDNS hervorgegangen ist und bis zu 1,2 Kilopaare Basen enthält und eine Nukleotidsequenz enthält, die für das Protein VP1 des Poliovirus oder für den Teil dieses Proteins kodiert, das für diejenige oder diejenigen der antigenen Determinanten kodiert, die als im wesentlichen verantwortlich für die Immunogenität oder die immunologische Spezifität des Proteins VP1 des Poliovirus angesehen werden können, d. h. die Fähigkeit des Proteins oder des Teils dieses Proteins, Antigen-Antikörper-Komplexe oder neutralisierende mono- oder polyklonale Seren zu bilden, hergestellt durch Injektion des gesamten an Formol gebundenen Poliovirus in ein Tier (Serum der Spezifität D).

2. Rekombinationsvektor nach Anspruch 1, dadurch gekennzeichnet, daß das oben genannten Fragment die Nukleotidsequenz enthält, die durch zwei PstI-Stellen begrenzt ist und sich von der Position 2 243 bis zur Position 3 417 der für ein Poliovirus des Typs PV-1 kodierenden cDNS erstreckt.

3. Rekombinationsvektor nach Anspruch 1, dadurch gekennzeichnet, daß das oben genannte DNS-Fragment die Nukleotidsequenz enthält, die sich von der Position 2 248 bis zur Position 3 421 der für ein Poliovirus vom Typ PV-1 kodierenden cDNS erstreckt.

4. Rekombinationsvektor nach Anspruch 1, dadurch gekennzeichnet, daß das oben genannte DNS-Fragment die Nukleotidsequenz enthält, die von den beiden äußeren Stellen HaeII und PstI begrenzt wird und sich von der Position 2 472 bis zur Position 3 421 der für ein Poliovirus vom Typ PV-1 kodierenden cDNS erstreckt.

5. Rekombinationsvektor nach Anspruch 1, dadurch gekennzeichnet, daß das oben genannte DNS-Fragment die Nukleotidsequenz enthält, die sich von der Position 2 480 bis zur Position 3 385 der für ein Poliovirus vom Typ PV1 kodierenden cDNS erstreckt.

6. Rekombinationsvektor nach Anspruch 5, dadurch gekennzeichnet, daß der Promotor auf die DNS von SV40 zurückgeht.

7. Expressionsprodukt des Inserts, erzeugt von einem durch den Vektor nach Anspruch 6 transformierbaren Mikroorganismus.

8. Impf-Wirkstoff, der das Expressionsprodukt nach Anspruch 7 enthält, gekoppelt an ein natürliches Protein oder an ein synthetisches Polypeptid mit einem ausreichenden Molekulargewicht, daß das gebildete Konjugat in der Lage ist, in vivo die Erzeugung von Antipoliovirus-Antikörpern einzuleiten.

# Fig.1.

```
                                                                2480      2490      2500
                                                                GGGTTAGGTCAGATGCTTGAA
                                                                   ⌐
                                                                 VP3 VP1

      2510      2520      2530      2540      2550      2560      2570      2580      2590      2600
AGCATGATTGACAACACAGTCCGTGAAACGGTGGGGGCGGCAACA[TCTAGA]GACGCTCTCCCAAACACTGAAGCCAGTGGACCAACACACTCCAAGGAAA
                                             ^
                                           XBA1

      2610      2620      2630      2640      2650      2660     ·2670      2680      2690      2700
TTCCGGCACTCACCGCAGTGGAAACTGGGGCCACAAATCCACTAGTCCCTTCTGATACAGTGCAAACCAGACATGTTGTACAACATAGGTCAAGGTCAGA
^                                        ^                                          ^
HPA11                                    HAE111                                     RSA1

      2710      2720      2730      2740      2750      2760      2770      2780      2790      2800
GTCTAGCATAGAGTCTTTCTTCGCGCGGGGTGCATGCGTGACCATTATGACCGTGGATAACCCAGCTTCCACCACGAATAAGGATAAGCTATTTGCAGTG
              ^^^                                            ^                      ^
              BCER                                          ALU1                   ALU1
              HHA1
              BCER

      2810      2820      2830      2840      2850      2860      2870      2880      2890      2900
TGGAAGATCACTTATAAAGATACTGTCCAGTTACGGAGGAAATTGGAGTTCTTCACCTAT[TCTAGA]TTTGATATGGAACTTACCTTTGTGGTTACTGCAA
^                                                            ^
SAU3A                                                       XBA1

      2910      2920      2930      2940      2950      2960      2970      2980      2990      3000
ATTTCACTGAGACTAACAATGGGCATGCCTTAAATCAAGTGTACCAAATTATGTACGTACCACC[AGGCGCT]CCAGTGCCCGAGAAATGGGACGACTACAC
                                 ^               ^   ^            ^**     ^
                                 RSA1            RSA1            HAE11    AVA1
                                                 RSA1            HHA1

      3010      3020      3030      3040      3050      3060      3070      3080      3090      3100
ATGGCAAACCTCATCAAATCCATCAATCTTTTACACCTACGGAACAGCTCCAGCCCGGATCTC[GGTACC]GTATGTTGGTATTTCGAACGCCTATTCACAC
                                         ^        ^ ^     ^^                        ^^
                                        ALU1     HPA11  KPN1                       ASU11
                                                 SAU3A  RSA1                       TAQ1

      3110      3120      3130      3140      3150      3160      3170      3180      3190      3200
TTTTACGACGGTTTTTCCAAAGTACCACTGAAGGACCAGTCGGCAGCACTAGGTGACTCCCTTTATGGTGCAGCATCTCTAAATGACTTCGGTATTTTGG
         ^
        RSA1
```

0 086 707

# Fig.2.

```
        3210        3220        3230        3240        3250        3260        3270        3280        3290        3300
CTGTTAGAGTAGTCAATGATCACAACCCGACCAAGGTCACCTCCAAAATCAGAGTGTATCTAAAACCCAAACACATCAGAGTCTGGTGCCCGCGTCCACC
                  ^^                                                                                  ^
                  BCL1                                                                                BCER
                  SAU3A

        3310        3320        3330        3340        3350        3360        3370        3380
GAGGGCAGTGGCGTACTACGGCCCTGGAGTGGATTACAAGGATGGTACGCTTACACCCCTCTCCACCAAGGATCTGACCACATAT
              ^           ^                         ^                         ^
              RSA1      HAE111                    RSA1                      SAU3A              VP1
```

# Fig.3.

```
            2250      2260      2270      2280      2290      2300
      CTGCAGTCCTCATGTACTATGGTAGTGCCATGGATTAGCAACACCACGTATCGGCAAA
      ^               ^
     PST1            RSA1

 2310      2320      2330      2340      2350      2360      2370      2380      2390      2400
CCATAGATGATAGTTTCACCGAAGGCGGATACATCAGCGTCTTCTACCAAACTAGAATAGTCGTCCCTCTTTCGACACCCAGAGAGATGGACATCCTTGG
                                                                        ^
                                                                       TAQ1

 2410      2420      2430      2440      2450      2460      2470↓     2480      2490      2500
TTTTGTGTCAGCGTGTAATGACTTCAGCGTGCGCTTGTTGCGAGATACCACACATATAGAGCAAAAGCGCTAGCACAGGGGTTAGGTCAGATGCTTGAA
                     ^                                            ^
                    HHA1                                        HAE11          VP3  VP1
                                                                HHA1

 2510      2520      2530      2540      2550      2560      2570      2580      2590      2600
AGCATGATTGACAACACAGTCCGTGAAACGGTGGGGGCGGCAACATCTAGAGACGCTCTCCCAAACACTGAAGCCAGTGGACCAACACACTCCAAGGAAA
                                             ^
                                            XBA1

 2610      2620      2630      2640      2650      2660      2670      2680      2690      2700
TTCCGGCACTCACCGCAGTGGAAACTGGGGCCACAAATCCACTAGTCCCTTCTGATACAGTGCAAACCAGACATGTTGTACAACATAGGTCAAGGTCAGA
^                             ^                                                  ^
HPA11                        HAE111                                            RSA1

 2710      2720      2730      2740      2750      2760      2770      2780      2790      2800
GTCTAGCATAGAGTCTTTCTTCGCGCGGGGTGCATGCGTGACCATTATGACCGTGGATAACCCAGCTTCCACCACGAATAAGGATAAGCTATTTGCAGTG
                    ^^^                                        ^                           ^
                    BCER                                      ALU1                        ALU1
                    HHA1
                    BCER

 2810      2820      2830      2840      2850      2860      2870      2880      2890      2900
TGGAAGATCACTTATAAAGATACTGTCCAGTTACGGAGGAAATTGGAGTTCTTCACCTATTCTAGATTTGATATGGAACTTACCTTTGTGGTTACTGCAA
^                                                      ^
SAU3A                                                 XBA1
```

# Fig.4.

```
       2910        2920        2930        2940        2950        2960        2970        2980        2990        3000
ATTTCACTGAGACTAACAATGGGCATGCCTTAAATCAAGTGTACCAAATTATGTACGTACCACCAGGCGCTCCAGTGCCCGAGAAATGGGACGACTACAC
                                  ^                        ^      ^            ^^                 ^
                                 RSA1                     RSA1   RSA1        HAE11             AVA1
                                                            RSA1              HHA1
```

```
       3010        3020        3030        3040        3050        3060        3070        3080        3090        3100
ATGGCAAACCTCATCAAATCCATCAATCTTTTACACCTACGGAACAGCTCCAGCCCGGATCTCGGTACCGTATGTTGGTATTTCGAACGCCTATTCACAC
                                            ^           ^  ^      ^^                        ^^
                                           ALU1       HPA11  KPN1                        ASU11
                                                      SAU3A RSA1                          TAQ1
```

```
       3110        3120        3130        3140        3150        3160        3170        3180        3190        3200
TTTTACGACGGTTTTTCCAAAGTACCACTGAAGGACCAGTCGGCAGCACTAGGTGACTCCCTTTATGGTGCAGCATCTCTAAATGACTTCGGTATTTTGG
                  ^
                 RSA1
```

```
       3210        3220        3230        3240        3250        3260        3270        3280        3290        3300
CTGTTAGAGTAGTCAATGATCACAACCCGACCAAGGTCACCTCCAAAATCAGAGTGTATCTAAAACCCAAACACATCAGAGTCTGGTGCCCGCGTCCACC
                  ^^                                                                                  ^
                 BCL1                                                                              BCER
                 SAU3A
```

```
       3310        3320        3330        3340        3350        3360        3370        3380        3390        3400
GAGGGCAGTGGCGTACTACGGCCCTGGAGTGGATTACAAGGATGGTACGCTTACACCCCTCTCCACCAAGGATCTGACCACATATGGATTCGGACACCAA
           ^     ^                          ^                              ^         |
          RSA1  HAE111                      RSA1                           SAU3A    VP1
```

```
       3410        3420
AACAAAGCGGTGTACACTGCAGG
           ^     ^
          RSA1  PST1
```

Fig.5a.

Fig.5b.

Fig.5c.

Fig.5d.

Fig.5e.

Fig.5f.

Fig.5g.

Fig.5h.

Fig.6a.

Fig.6b.

Fig.6c.

Fig.6d.

Fig.6e.

Fig.6f.